# EUROPEAN PATENT APPLICATION

(11) **EP 1 752 159 A1**
(43) Date of publication of application: **14.02.2007**
(21) Application number: 05741471.6
(22) Date of filing: 17.05.2005
(51) Int. Cl.: A61K 38/00, A61P 11/00, A61P 29/00, A61P 31/04

(54) **MEDICINAL COMPOSITION AND THERAPEUTIC METHOD**

(30) Priority: 17.05.2004 JP 2004145971
(71) Applicant: Keio University, Tokyo 108-8345 (JP)
(72) Inventor: AIKAWA, Naoki, c/o Keio Univ. School of Medicine, Tokyo 160-8582 (JP); FUJISHIMA, Seitaro, Keio Univ. School of Medicine, Tokyo 160-8582 (JP); SEKINE, Kazuhiko, Keio Univ. School of Medicine, Tokyo 160-8582 (JP)
(74) Representative: Jones, Helen M.M.
(86) International application number: PCT/JP2005/008944
(87) International publication number: WO 2005/110450

(57) **Abstract**

The object of the present invention is, based on the finding of a novel effect of IL-18, to provide pharmaceutical compositions containing IL-18 and therapeutic methods, for preventing, improving, or treating inflammatory diseases such as systemic inflammatory response syndrome (SIRS). Inflammatory diseases, such as SIRS, can be prevented, improved, or treated by administering the pharmaceutical compositions according to the present invention containing IL-18 in an amount which does not bring the decreased interleukin 18 level in either plasma or liver to a higher level than normal.

## Description

### CROSS REFERENCE TO RELATED APPLICATIONS

This application claims the benefit of priority to a Japanese Patent Application No. 2004-145971, filed on May 17, 2004, which is incorporated herein by reference.

### TECHNICL FIELD

The present invention relates to pharmaceutical compositions and therapeutic methods for preventing, improving or treating inflammatory diseases.

### BACKGROUND ART

Interleukin 18 (IL-18) was identified as an interferon γ (IFN-γ)-inducing factor, which appears in the blood when a small dose of lypopolysaccharide (LPS) has been intravenously injected into a mouse pretreated with heat-killed Propionibacterium acnes (P. acnes), a gram-positive bacillus. IFN-γ enhances cellular immunity by activating macrophages and NK cells, thereby elevating resistance to infection with pathogens such as bacteria. At present, it has been clarified that IL-18 induces IFN-γ production by activating CD8 cells, NK cells, and TH-1 cells.

IL-18, unlike many cytokines, is observed to be expressed at a low level even under normal conditions. However, its expression is enhanced upon infection with pathogens (viruses, prokaryotes, or eukaryotes), thereby conferring cellular resistance to pathogens. For example, administration of IL-18 into mice prior to infection with HSV remarkably improves the survival rate of the mice. Administration of IL-18 also suppresses proliferation of a fungus Cryptococcus neoformans in cryptococcosis, thereby conferring resistance to Candida infection. Further, treatment of a mouse with anti-IL-18 antibody impairs its resistance to Salmonella typhimurim, an intracellular microorganism, whereas administration of IL-18 to a mouse infected with a toxic strain of Salmonella at a lethal dose decreases the number of the bacteria in the tissue, thereby reducing lethality ("Cytokine Reference : A Compendium of Cytokines and Other Mediators of Host Defense, edited by Joost J. Oppenheim, Marc Feldmann, and Scott K. Durum; Academic Press, U.S.A, 2000; pp. 338-350.). IL-18 is known to induce cytokines other than IFN-γ, and chemokines, among which are inflammatory cytokines such as IL-8, TNF, and IL-6. In addition, from the fact that IL-18-knockout mice have resistance to administration of LPS, which induces inflammatory cytokines, it is evident that IL-18 is a factor indispensable to LPS-induced pro-inflammatory reactions.

### DISCLOSURE OF THE INVENTION

### PROBLEMS TO BE SOLVED BY THE INVENTION

As described above, IL-18 is known to be a mediator of immune response and has been considered to play an important role in the induction of the inflammatory cytokines.

However, the inventors of the present application has discovered a novel effect of IL-18, i.e. suppression of inflammation. Accordingly, based on the finding of this novel effect of IL-18, the object of the present invention is to provide pharmaceutical compositions containing IL-18 and therapeutic methods for preventing, improving or treating inflammatory diseases such as systemic inflammatory response syndrome (SIRS).

### MEANS FOR SOLVING THE PROBLEMS

IL-18 is observed to be expressed at a low level even under normal conditions. The inventors found that severe burn injury decreases the IL-18 expression level, and that administration of a small dose of IL-18 to severely burned mice remarkably improves the survival rate. The inventors further found that, although splenic cells isolated from those burned mice secrete inflammatory cytokine in response to LPS under culture condition, this secretion is suppressed in the IL-18-administered mice, and thus accomplished the present invention.

The pharmaceutical composition according to the present invention is a pharmaceutical composition for preventing, improving or treating an inflammatory disease, which contains interleukin 18 (IL-18). The inflammatory disease may be SIRS, sepsis syndrome, or septicemia. The inflammatory disease may be acute lung injury or multi organ dysfunction syndrome, secondary to SIRS. The inflammatory disease may act independently of interferon γ.

The inflammatory disease to be treated by the pharmaceutical composition may decrease the interleukin 18 level in the liver. In this case, the pharmaceutical composition according to the present invention preferably does not bring the liver interleukin 18 level which has decreased in the aforementioned inflammatory disease to a higher level than normal. "Normal" as used herein refers to a measured value in a healthy individual, which typically has a certain extent of a range.

In the therapeutic method according to the present invention, an effective dose of IL-18 is administered to a patient suffering from an inflammatory disease. The inflammatory diseases may be SIRS, sepsis syndrome, or septicemia. The inflammatory disease may be acute lung injury or multi organ dysfunction syndrome, secondary to SIRS. The inflammatory disease may act independently of interferon γ.

Further, the inflammatory disease to be treated by the pharmaceutical composition may be a disease which decreases interleukin 18 level in plasma or liver. In this case, the pharmaceutical composition preferably does not to bring the interleukin 18 level in plasma or liver which has decreased in the inflammatory disease to a higher level than normal.

The therapeutic method according to the present invention is a therapeutic method for treating a human or nonhuman vertebrate individual suffering from an inflammatory disease, including administering a pharmaceutical composition containing interleukin 18 to the vertebrate individual. The inflammatory disease may decrease interleukin 18 level in plasma or liver. It is preferable to administer the pharmaceutical composition containing interleukin 18 in an amount which does not bring the interleukin 18 level in plasma or liver which has decreased in the aforementioned inflammatory disease to a higher level than normal.

### BRIEF DESCRIPTION OF THE DRAWINGS

FIG. 1 is a graph showing a time course of the IL-18 content in livers and lungs after burn injury in the mice of the control group and the burn group in an embodiment of the present invention.
FIG. 2 is a graph showing a time course of the plasma IL-18 level after burn injury in humans in an embodiment of the present invention.
FIG. 3 is a graph showing the survival rates of the mice of the control group, the non-IL-18-administered group, and the IL-18-administered group after LPS administration in an embodiment of the present invention.
FIG. 4 is a graph showing images of the histological sections of lungs after the LPS administration into the mice of the control group, the non-IL-18-administered group, and the IL-18-achninistered group in an embodiment of the present invention.
FIG. 5 shows the secreted amounts of various cytokines in the splenic cells isolated from the mice of the control group, the non-IL-18-administered group, and the IL-18-administered group, which were stimulated with anti-CD3 antibody in vitro in an embodiment of the present invention.

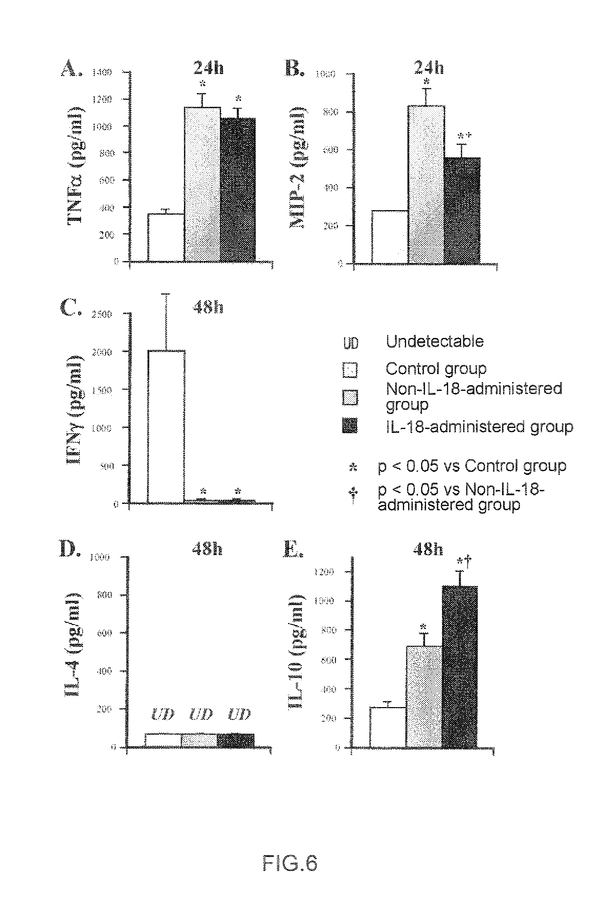

FIG. 6 shows the secreted amounts of various cytokines in the splenic cells isolated from the mice of the control group, the non-IL-18-administered group, and the IL-18-administered group, which were stimulated with LPS in vitro in an embodiment of the present invention.
FIG. 7 shows the secreted amounts of cytokines in the lungs of the mice of the control group, the untreated group, and the IL-18 administrated group in an embodiment of the present invention.

### PREFERRED EMBODIMENT OF THE INVENTION

Embodiments of the present invention accomplished based on the above-described findings are hereinafter described in detail by giving examples. Unless otherwise explained, methods described in standard sets of protocols such as J. Sambrook and E. F. Fritsch & T. Maniatis (Ed.), "Molecular Cloning, a Laboratory Manual (3rd edition), Cold Spring Harbor Press and Cold Spring Harbor, New York (2001); and F. M. Ausubel, R. Brent R. E. Kingston, D. D. Moore, J. G. Seidman, J. A. Smith, and K. Struh1 (Ed.),"Current Protocols in Molecular Biology," John Wiley & Sons Ltd., or alternatively, modified/changed methods from these are used. When using commercial reagent kits and measuring apparatus, unless otherwise explained, attached protocols to them are used.

The object, characteristics, and advantages of the present invention as well as the ideas thereof will be apparent to those skilled in the art from the descriptions given herein. It is to be understood that the embodiments and specific examples of the invention described herein below are to be taken as preferred examples of the present invention. These descriptions are for illustrative and explanatory purposes only and are not intended to limit the invention to these embodiments or examples. It is further apparent to those skilled in the art that various changes and modifications may be made based on the descriptions given herein within the intent and scope of the present invention disclosed herein.

### === Diseases to be dealt with the present invention ===

The pharmaceutical composition according to the present invention is a pharmaceutical composition containing IL-18 for preventing, improving or treating an inflammatory disease.

The inflammatory disease is exemplified by systemic inflammatory response syndrome (SIRS). SIRS is a concept of inflammatory response put forward by a collaboration of the American College of Chest Physicians and the Society of Critical Care Medicine in 1991, and defined to be diagnosed when two or more of the following four conditions are present: 1) body temperature is 38°C or higher, or 36°C or lower; 2) heart rate is 90 beats/min or more; 3) respiratory rate is 20 breaths/min, or PaCO₂ is less than 32 torr; and 4) leukocyte count is 12,000 cells/mm³ or more, or 4,000 cells/mm³ or less, or immature leukocytes are 10% or more. SIRS is categorized into the systemic inflammatory conditions upon trauma, burns, pancreatitis, highly invasive surgery, etc., which are not accompanied by infection, and the systemic inflammatory conditions accompanied by infection (sepsis syndrome). The sepsis syndromes are further categorized into the septicemia, a condition accompanied by the infection by bacteria in the bloodstream, and other conditions. All these inflammatory diseases can be considered to be treated with the pharmaceutical composition according to the present invention.

For example, upon severe burn injury, the affected area is infected with bacteria and the multiplied bacteria invade the bloodstream, thereby causing septicemia. The septicemia results in acute lung injury (ALI), which can sometimes lead to multi organ dysfunction syndrome (MODS). The pharmaceutical composition according to the present invention can treat such severe injury as well by exerting an anti-inflammatory effect.

The studies by the present inventors have revealed that severe burn injury, septicemia, acute lung injury, etc is accompanied with a decrease in the level of IL-18 in plasma or liver. An administration of IL-18 against these diseases does not increase the level of IFN-γ that is secreted from splenic cells in response to inflammatory stimuli. Therefore, it is considered that IL-18 administration against these diseases has the effect of suppressing inflammation independently of IFN-γ. However, excessive doses may induce IFN-γ, therefore it is preferable to administer the IL-18 so as not to bring the IL-18 level to a higher level than the normal IL-18 level.

### === Dosage form and dosage regimen of the pharmaceuticals according to the present invention ===

When administered to a human, IL-18 or its pharmacologically acceptable salt may be administered intravenously at a dose of, for example, about 8 mg/kg (body weight) daily, in a single dose or divided doses, and the dose and the number of administration may be suitably changed depending on symptoms, age, dosage regimen, etc.

IL-18 and its pharmacologically acceptable salts may be orally administered in preparations such as tablets, capsules, granule, powder, syrups, ointments, etc.; directly applied to the affected area; or parenterally administered by intraperitoneal, intravenous, intra-arterial, intramuscular, subcutaneous or other injections, in preparations such as injectable formulations, suppositories, etc. Alternatively, it may be applied or sprayed to the skin, mucosa, the affected area, etc., in liquid form, or may be inhaled from the nasal mucosa, throat mucosa, etc.

The content of IL-18 or its pharmacologically acceptable salt (an active ingredient) in a preparation may vary between 1 to 90% by weight. For example, when the preparation is in the form of a tablet, a capsule, a granule, a powder, etc., the content of the active ingredient is preferably 5 to 80% by weight. In the case of a liquid preparation such as syrup, the content of the active ingredient is preferably 1 to 30% by weight. In the case of an injectable preparation for the parenteral administration, the content of the active ingredient is preferably 1 to 10% by weight.

IL-18 and its pharmacologically acceptable salts may be formulated by known methods by using formulation additives such as: excipients (sugars such as lactose, sucrose, glucose, and mannitol; starches such as potato, wheat, and corn; inorganic substances such as calcium carbonate, calcium sulfate, and sodium hydrocarbonate; cellulose crystal; etc.), binders (starch-paste liquid, gum arabic, gelatin, sodium alginate, methylcellulose, ethylcellulose, polyvinylpyrrolidone, polyvinyl alcohol, hydroxypropylcellulose, carmellose, etc.), lubricants (magnesium stearate, talc, hydrogenated vegetable oil, macrogol, and silicone oil), disintegrators (starch, agar, gelatin powder, cellulose crystal, sodium carboxymethylcellulose, calcium carboxymethylcellulose, calcium carbonate, sodium bicarbonate, sodium alginate, etc.), correctives (lactose, sucrose, glucose, mannitol, fragrant essential oils, etc.), solvents (injectable water, sterile purified water, sesame oil, soybean oil, corn oil, olive oil, cottonseed oil, etc.), stabilizers (inert gases such as nitrogen and carbon dioxide; chelating agents such as EDTA and thioglycolic acid; reducing substances such as sodium bisulfite, sodium thiosulfate, L-ascorbic acid, rongalite, etc.), preservatives (paraoxybenzoate esters, chlorobutanol, benzyl alcohol, phenol, benzalkonium chloride, etc.), detergents (hydrogenated castor oil, polysorbates 80 and 20, etc.), buffers (sodium salts of citric acid, acetic acid, and phosphoric acid; boric acid; etc.), and diluents.

It should be noted that examples of pharmacologically acceptable salts of IL-18 that may be used according to the present invention include, but not limited to, organic salts such as quaternary ammonium salts, and metal salts such as alkali metals, etc.

### EXAMPLES

To confirm immune response to inflammatory diseases resulted from burn, as well as effects of TL-18 administration in humans and nonhuman vertebrates, the following experiments were performed.

### <Example 1> Preparation of a mouse model of burn injury

Seven-week-old male Balb/c mice (body weight 23 to 28 g) were caged and supplied with water and food ad libitum for about a week, and then the mice were divided into a control group and a burn group. The mice were shelved over the dorsum under pentobarbital anesthesia (50 mg/kg) one day before the experiment (Day 0). On the next day (day 1), all mice were anesthetized with ether. For the mice of the burn group, their shelved dorsal area was exposed to hot steam for 5 seconds to cause full-thickness burn covering about 15% of total body surface area. Control mice were not exposed to hot steam. All the mice were immediately resuscitated with 4 ml of physiological saline.

### <Example 2> Measurement of IL-18 and IL-12 levels in lung and liver

IL-12 as well as IL-18 is the cytokine classified as Th-1 cytokines involved in Th-1 cell differentiation. To examine the behaviors of these Th-1 cytokines after burn injury, the IL-18 and IL-12 contents in lung and liver of the mice in the burn group and the control group prepared according to Example 1 were measured.

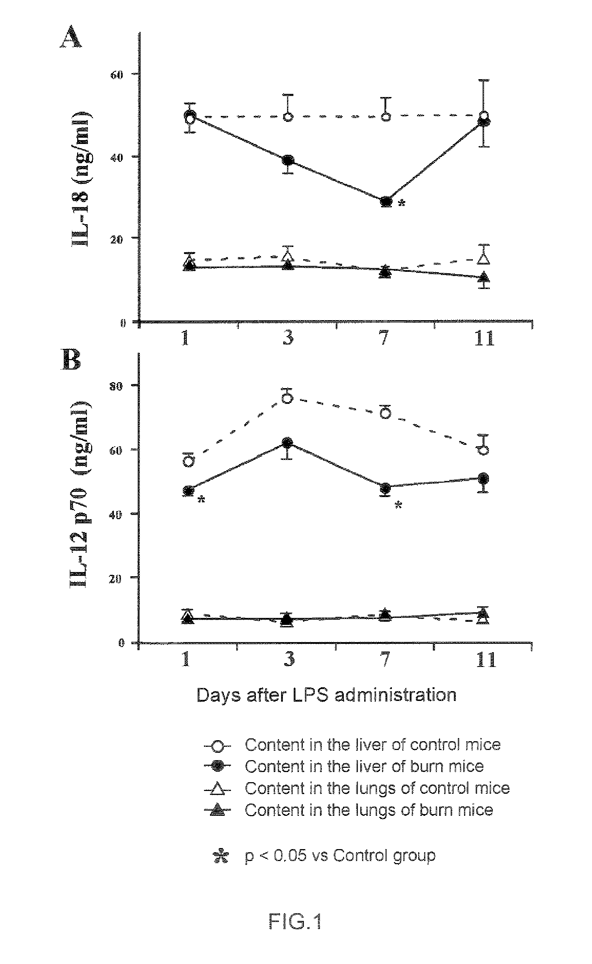

Twenty-four hours after the burn treatment, mice were anesthetized by intraperitoneal injection of pentobarbital, and their lungs and livers were resected. The resected lungs and livers were homogenized with 10 or 50 times by weight of phosphate buffered saline (PBS), centrifuged, and the supernatants were removed. Cytokine levels in the supernatants of lungs and livers were determined for IL-18 and IL-12 by the sandwich ELISA method using an ELISA kit (BD Biosciences). The results are shown in FIG. 1. The cytokine levels were compared by an analysis of variance (ANOVA), followed by a Scheffe's comparison test (A significant difference was judged by p < 0.05. The levels of the following cytokines were compared in the same manner.).

The Th-1 cytokine levels in the lung were significantly lower than those in the liver. In the liver of the burn group mice, the IL-18 content remarkably decreased on day 7, and the IL-12 level was generally lower until day 11. These findings revealed that, after severe burn injury, the Th-1 cytokine level decreased in the liver.

### <Example 3> Measurement of IL-18 levels after burn injury in humans

To observe changes in the IL-18 level after burn injury in humans, plasma IL-18 levels of the severely-burned human subjects were measured at some time points after burn injury, and the results were divided into four subgroups: day 1, day 2 to 3, day 6 to 8, and day 9 to 11, after the burn injury. The measurements were performed by using the ELISA kit (BD Biosciences) as in Example 2. Mean values for each subgroup were calculated from the plurality of measurements of the IL-18 levels in each subgroup. The IL-18 level in each subgroup was subjected to an analysis of variance by using these mean values, and then statistically treated by Fisher's PLSD method (a significant difference was judged by p < 0.05).

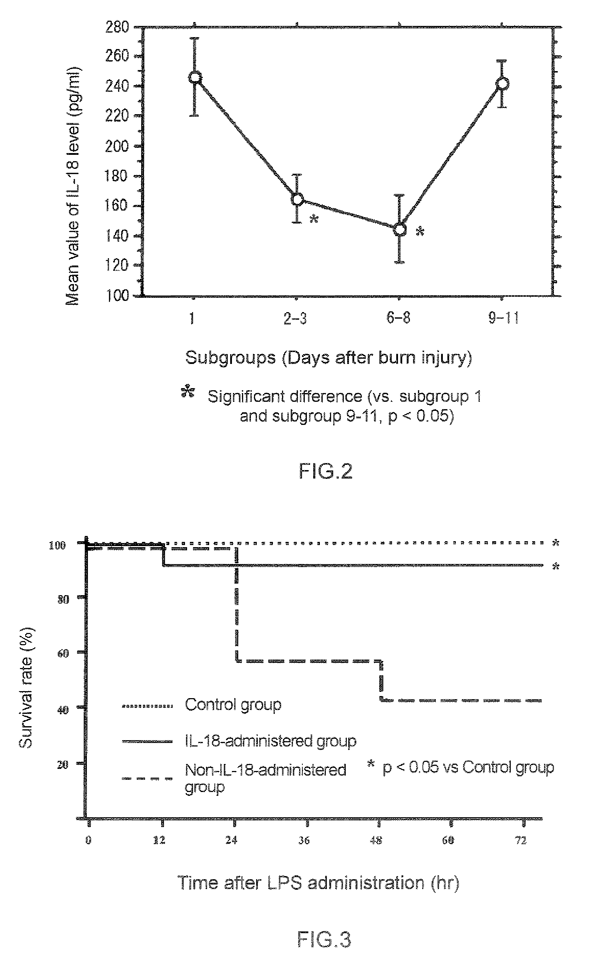

As shown in FIG. 2, the IL-18 levels showed a significant decrease on day 2 to 3 after the burn injury, and this decrease reached its peak on day 6 to 8. Such behavior of the IL-18 level is the same as the changes in the IL-18 content in the liver of the burn group mice described in Example 2. It was thus confirmed that the IL-18 level decreased after burn injury in humans as well.

### <Example 4> Effect of IL-18 administration on survival rate after endotoxin treatment

The results from Examples 2 and 3 suggested that the decrease in the Th-1 cytokine levels after burn injury is critically involved in inflammatory diseases such as septicemia and acute lung injury resulted from severe burn, etc. Thus, by using an inflammation model mouse that has developed acute inflammation due to administration of endotoxin (LPS) after burn injury, the effect of administering a small dose of IL-18 was examined on the survival rate after the LPS administration as a marker.

Mice in the burn group prepared according to Example 1 were divided into two groups: the IL-18-administered group and the non-IL-18-administered group. The mice in the IL-18 administered group received 0.2 µg of recombinant IL-18 (MBL Co. Ltd.) every other day for 10 days after the burn injury, whereas mice in the non-IL-18-administered group received nothing. On the eleventh day (day 12) after the burn treatment, inflammation was induced in all the mice of the three groups by intravenous injections of 10 ml/kg body weight of physiological saline containing 3 mg/kg body weight of LPS (Escherichia coli 0111:B4 endotoxin; Sigma Corp.). Then, the number of mice survived in each group was examined at every 12 hours until 72 hours after the induction. FIG. 3 is a graph showing the results. The survival rates were compared between the groups by using the Wilcoxon-Gehan test (a significant difference was judged by p < 0.05).

At 72 hours after the burn, the survival rate of the control group was 100%, and the rate of the non-IL-18-administered group was 42.9% whereas the rate of the IL-18-administered group was 91.7%, indicating that while the survival rate of the non-IL-18 administered group is considerably lower compared with that for the control group, the IL-18 administration remarkably improves the survival rate.

These results indicate that IL-18 has an anti-inflammatory effect, suggesting that it is useful for a pharmaceutical composition for preventing, improving, or treating inflammatory diseases, such as systemic inflammatory response syndromes, including septicemia, etc.

### <Example 5> Effect of IL-18 administration on pulmonary edema

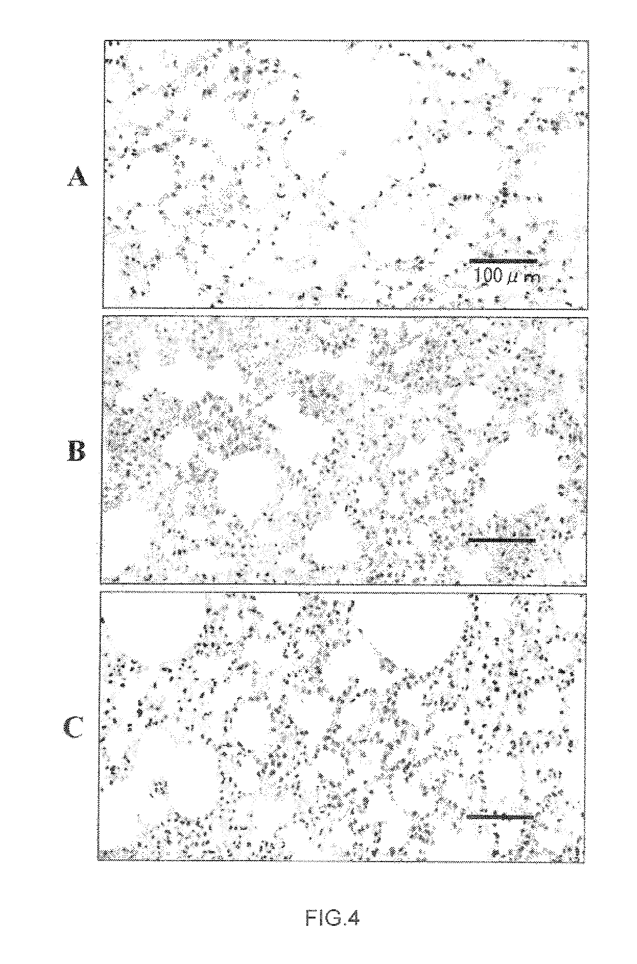

Using the inflammation model mice described in Example 4, their lungs were resected, and their upper lobes were fixed with 4% formaldehyde solution, embedded in paraffin, sectioned, and histopathogically examined. As shown in FIG. 4, unlike the control group (FIG. 4A), in the non-IL-18-administered group (FIG. 4B) a severe pulmonary edema was induced, and the number of neutrophils increased. Being a pathological condition similar to that in acute lung injury, this can be a model of the acute lung injury. Meanwhile, in the IL-18-administrated group (FIG. 4C), improvements of conditions such as pulmonary edema and the increased number of neutrophils were observed.

It is thus suggested that the administration of IL-18 is useful for preventing, improving, or treating diseases such as acute lung injury and multi organ dysfunction syndrome, secondary to SIRS, etc.

### <Example 6> Cytokine secretion by activated splenic cells

In this Example, the amount of secreted cytokine was measured by isolating splenic cells from the inflammation model mice in Example 4 and then activating the splenic cells by administering anti-CD3 antibody or LPS under the culture condition. A stimulation of splenic cells with anti-CD3 antibody activates immune response mediated by T cells, and a stimulation of splenic cells with LPS activates mainly macrophages, resulting in secretion of inflammatory cytokines.

First, the resected spleens were gently minced in RPMI 1640 supplemented with 5% fetal calf serum (FCS), and filtered through nylon mesh strainer. The splenic cells were treated with 0.84% ammonium chloride hemolyzing solution to hemolyze contaminated red blood cells. After centrifugation, the splenic cells were resuspended in PRMI-1640/5% FCS, added with 1 µg/mL anti-CD3 antibody (mAb clone I45-2C11, BD Biosciences) or 10 µg/mL LPS, and incubated in 5% CO₂ at 37°C for 24 or 48 hours. For the culture supernatant, the levels of the inflammatory cytokines TNF-α and MIP-2, the Th-1 cytokine IFN-γ, and the Th-2 cytokines IL-4 and IL-10, were determined by using ELISA kits (BD Biosciences).

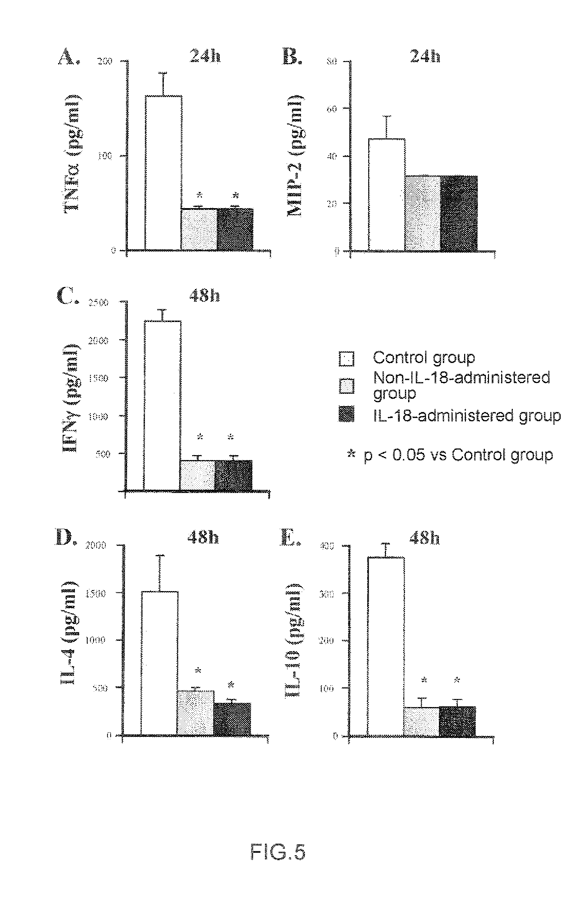

When the splenic cells were stimulated with anti-CD3 antibody (FIG. 5), unlike the control group, the secreted amounts of the inflammatory cytokines, the Th-1 cytokine, and the Th-2 cytokines all decreased. The secreted amounts of these cytokines in the IL-18-administered group also decreased. Taken together, it is evident that IL-18 cannot contribute to the decrease in cytokine secretion by T cell activation.

In contrast, when the spleen cells were stimulated with LPS, the secreted amounts of the inflammatory cytokines increased, whereas the secreted amount of the Th-1 cytokine markedly decreased, and the secreted amount of IL-10, a Th-2 cytokine, increased (IL-4 secretion was undetectable in either case). In the IL-18-administered group, unlike the non-IL-18-administered group, the secretion of MIP-2, one of the inflammatory cytokines, decreased, and that of IL-10, one of the Th-2 cytokines, increased compared with the non-IL-18-administered group. This indicates that IL-18 has an anti-inflammatory effect at the cytokine level. Further, from the fact that the amount of IFN-γ secretion did not increased, it is considered that the effect of IL-18 administration on individual mice does not depend on IFN-γ, and has a different mechanism from the known effects of IL-18 when IL-18 is enhanced against pathogenic infection.

### <Example 7> Cytokine secretion after endotoxin treatment

The results of Example 4 revealed that the IL-18 administration markedly improves survival rates of the inflammation model mice. Thus, the following experiment was performed to examine the relationship between IL-18 and the secreted amounts of cytokines in inflammation model mice.

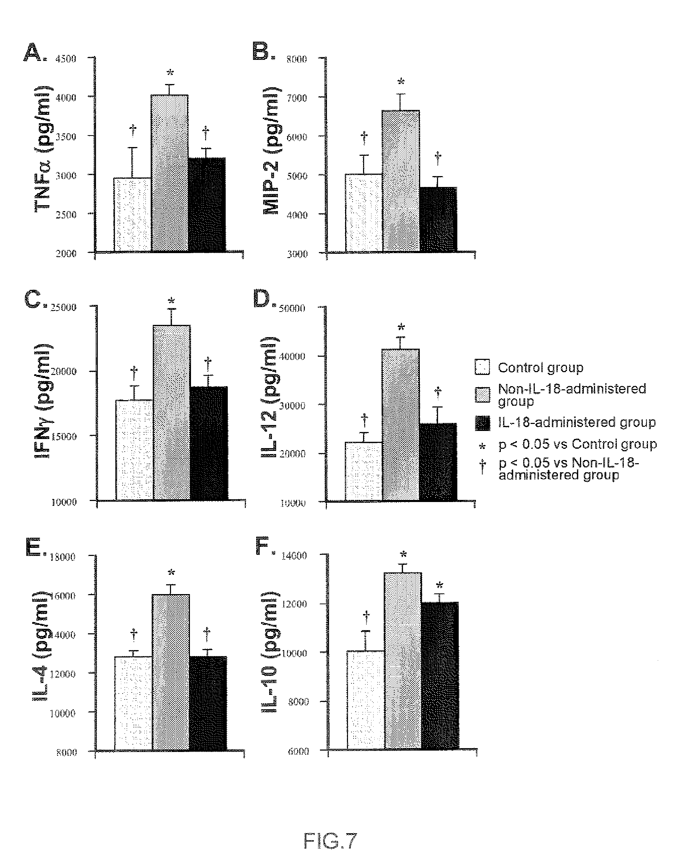

In this Example, the lungs of the inflammation model mice were isolated at 12 hours after the LPS administration, homogenized with 10 or 50 times by weight of phosphate buffered saline (PBS), and centrifuged, and the supernatants were removed. The levels of TNFα, IFN-γ, IL-4, IL-10, IL-12, IL-18, and MIP-2 in the lung supernatants were determined by the sandwich ELISA method using ELISA kits (BD Biosciences or Wako Pure Chemical Industries, Ltd.). The results are shown in FIG. 7. Cytokine levels were compared by an analysis of variance (ANOVA), followed by a Scheffe's comparison test (a significant difference was judged by p < 0.05).

As shown in FIG. 7, the secreted amounts of the inflammatory cytokines, the Th-1 cytokine, and the Th-2 cytokines in the non-IL-18-administered group all markedly increased compared with the control group, whereas in the IL-18-administered group, the secreted amounts of the inflammatory cytokines (TNFα and MIP-2) significantly decreased. Therefore, this indicates that IL-18 has an anti-inflammatory effect at the cytokine level.

### INDUSTRIAL APPLICABILITY

The present invention provides pharmaceutical compositions containing IL-18 for preventing, improving or treating inflammatory diseases such as SIRS.

## Claims

1. A pharmaceutical composition for preventing, improving or treating an inflammatory disease, comprising interleukin 18 (IL-18).

2. The pharmaceutical composition of claim 1, wherein the inflammatory disease is systemic inflammatory response syndrome (SIRS).

3. The pharmaceutical composition of claim 2, wherein the inflammatory disease is sepsis syndrome.

4. The pharmaceutical composition of claim 3, wherein the inflammatory disease is septicemia.

5. The pharmaceutical composition of claim 1, wherein the inflammatory disease is an acute lung injury or a multi organ dysfunction syndrome, secondary to SIRS.

6. The pharmaceutical composition of claim 1, wherein said composition acts independently of interferon γ (IFN-γ).

7. The pharmaceutical composition of claim 1, wherein the inflammatory disease decreases the interleukin 18 level in either plasma or liver.

8. The pharmaceutical composition of claim 7, wherein said composition does not bring the interleukin 18 level in either plasma or liver having decreased in the inflammatory disease to a higher level than normal.

9. A therapeutic method, for treating a non-human vertebrate individual suffering from an inflammatory disease, comprising administering a pharmaceutical composition comprising interleukin 18 to the vertebrate individual.

10. The therapeutic method of claim 9, wherein the inflammatory disease decreases the interleukin 18 level in either plasma or liver.

11. The therapeutic method of claim 10, comprising the step of administering the pharmaceutical composition comprising IL-18 in an amount which does not bring the interleukin 18 level in either plasma or liver having decreased in the inflammatory disease to a higher level than normal.
